# FASCICULE DE BREVET EUROPEEN

(11) **EP 1 002 120 B1**
(45) Date de publication et mention de la délivrance du brevet: **20.04.2005**
(21) Numéro de dépôt: 99920929.9
(22) Date de dépôt: 27.05.1999
(51) Int. Cl.: C12N 15/86, C12N 5/10, A61K 48/00

(54) **VECTEURS ADENOVIRAUX CHIMERES**
CHIMÄRE ADENOVIRALE VEKTOREN
CHIMERIC ADENOVIRAL VECTORS

(30) Priorité: 27.05.1998 FR 9806654
(43) Date de publication de la demande: 24.05.2000
(73) Titulaire: TRANSGENE S.A., 67000 Strasbourg (FR)
(72) Inventeur: MEHTALI, Majid, 1083 CJ Amsterdam (NL); LUSKY, Monika, D-07910 Freiburg (DE); WINTER, Arend, Jan, F-67100 Strasbourg (FR)
(74) Mandataire: Warcoin, Jacques
(86) Numéro de dépôt international: PCT/FR1999/001238
(87) Numéro de publication internationale: WO 1999/061638

(56) Documents cités:
- WO-A-97/45550
- FR-A- 2 707 664
- GHOSH-CHOUDHURY G. ET AL.: "HUMAN ADENOVIRUS CLONING VECTORS BASED ON INFECTIOUS BACTERIAL PLASMIDS" GENE, vol. 50, no. 1/03, 1 janvier 1986 (1986-01-01), pages 161-171, XP002004335
- REDDY ET AL.: "Nucleotide sequence, genome organization and transcription map of bovine adenovirus type 3" JOURNAL OF VIROLOGY, vol. 72, no. 2, février 1998 (1998-02), pages 1394-1402, XP002087289
- MITTAL ET AL.: "Development of a bovine adenovirus type 3-based expression vector" JOURNAL OF GENERAL VIROLOGY, vol. 76, 1995, pages 93-102, XP002087288

## Description

La présente invention concerne de nouveaux vecteurs adénoviraux qui présentent la caractéristique de contenir une région essentielle à l'encapsidation hétérologue par rapport au génome adénoviral dont ils dérivent. Ces vecteurs peuvent être utilisés à titre de vecteurs auxiliaires ou recombinants, les premiers permettant la propagation des seconds. L'invention a également pour objet un procédé pour préparer une préparation virale contenant lesdits vecteurs adénoviraux, une cellule, une composition pharmaceutique ou de matière les comprenant ainsi que leur utilisation à des fins thérapeutiques ou prophylactiques. Enfin, la présente invention a également trait à un génome adénoviral d'origine animale présentant des capacités d'encapsidation atténuées par rapport au génome natif dont il dérive. L'invention présente un intérêt tout particulier pour des perspectives de thérapie génique, notamment chez l'homme.

La thérapie génique se définit comme le transfert d'information génétique dans une cellule ou un organisme hôte. Le premier protocole appliqué à l'homme a été initié aux Etats Unis en septembre 1990 sur un patient génétiquement immunodéficient en raison d'une mutation affectant le gène codant pour l'Adénine Désaminase (ADA). Le succès relatif de cette première expérimentation a encouragé le développement de cette technologie pour diverses maladies aussi bien génétiques (dans le but de corriger le dysfonctionnement d'un gène défectueux) qu'acquises (cancers, maladies infectieuses comme le SIDA...). La plupart des stratégies actuelles utilisent des vecteurs pour véhiculer le gène thérapeutique vers sa cible cellulaire. De nombreux vecteurs tant viraux que synthétiques ont été développés au cours de ces dernières années et ont fait l'objet de nombreuses publications accessibles à l'homme du métier.

L'intérêt des adénovirus à titre de vecteurs de thérapie génique a déjà été évoqué dans de nombreux documents de l'art antérieur. Ils infectent de nombreux types cellulaires, aussi bien des cellules en division que quiescentes, sont non intégratifs et peu pathogènes. En outre, ils possédent un tropisme naturel pour les voies respiratoires. Ces propriétés particulières font des adénovirus des vecteurs de choix pour de nombreuses applications thérapeutiques et même vaccinales. A titre indicatif, leur génome est constitué d'une molécule d'ADN linéaire et bicaténaire d'environ 36 kb qui porte une trentaine de gènes intervenant dans le cycle viral. Les gènes précoces (E1 à E4 ; E pour early en anglais) sont répartis en 4 régions dispersées dans le génome. Les régions E1, E2 et E4 sont essentielles à la replication virale alors que la région E3 impliquée dans la modulation de la réponse immunitaire anti-adénovirus chez l'hôte ne l'est pas. Les gènes tardifs (L1 à L5 ; L pour late signifiant tardif en anglais) codent majoritairement pour les protéines de structure et recouvrent en partie les unités de transcription précoces. Ils sont pour la plupart transcrits à partir du promoteur majeur tardif MLP (pour Major Late Promoter en anglais). En outre, le génome adénoviral porte à ses extrémités des régions agissant *en cis* essentielles à l'encapsidation constituées de séquences terminales inversées (ITR) situées aux extrémités 5' et 3' et d'une région d'encapsidation qui suit l'ITR 5'.

Les vecteurs adénoviraux actuellement utilisés dans les protocoles de thérapie génique sont dépourvus de la majeure partie de la région E1 afin d'éviter leur dissémination dans l'environnement et l'organisme hôte. Des délétions supplémentaires dans la région E3 permettent d'accroître les capacités de clonage. Les gènes d'intérêt sont introduits dans l'ADN viral à la place de l'une ou l'autre région délétée. Si la faisabilité du transfert de gènes en utilisant ces vecteurs dits de première génération est maintenant bien établie, la question de leur innocuité reste posée. Outre le risque de générer des particules compétentes pour la replication, l'immunogénécité potentielle des protéines virales encore exprimées peut dans certaines applications particulières s'opposer à la persistance des cellules transduites et à l'expression stable du transgène. Ces inconvénients ont justifié la construction de vecteurs de nouvelles générations. Ils conservent les régions *en cis* (ITRs et séquences d'encapsidation) essentielles à l'encapsidation mais comportent des modifications génétiques supplémentaires visant à supprimer l'expression *in vivo* de l'essentiel des gènes viraux (voir par exemple la demande internationale WO94/28152). A cet égard, un vecteur dît minimal, déficient pour l'ensemble des fonctions adénovirales représente une alternative de choix.

Les techniques de préparation des vecteurs adénoviraux sont largement décrites dans la littérature. Dans un premier temps, le génome est constitué par recombinaison homologue dans la lignée 293 (voir notamment Graham et Prevect, 1991, Methods in Molecular Biology, Vol *7*, Gene Transfer and Expression Protocols ; Ed E. J. Murray, The Human Press Inc, Clinton, NJ) ou dans *Escherichia coli* (voir par exemple la demande internationale WO96/17070). Il est ensuite nécessaire de propager le vecteur afin de constituer un stock de particules virales le contenant. Cette étape de production est critique et doit permettre d'atteindre des titres élevés en particules infectieuses pour pouvoir envisager un développement à grande échelle en vue de la préparation de lots cliniques. On utilise à cet effet des lignées de complémentation fournissant *en trans* les produits d'expression viraux pour lesquels le vecteur est defectif. Par exemple, les virus délétés de E1 peuvent être propagés dans la lignée 293, établie à partir de cellules de rein embryonnaire humain (Graham et al., 1977, J. Gen. Virol. *36*, 59-72). Pour ce qui est des vecteurs de seconde génération, on peut avoir recours à des lignées complémentant deux fonctions virales essentielles, telles que celles décrites par Yeh et al. (1996, J. Virol. *70*, 559-565), Krougliak et Graham (1995, Human Gene Therapy *6*, 1575-1586), Wang et al. (1995 Gene Therapy *2*, 775-783), Lusky et al. (1998, J. Virol. *72*, 2022-2033) et dans les demandes internationales WO94/28152 et WO97/04119. Du fait de la toxicité potentielle des produits d'expression viraux, ces lignées nécessitent d'être optimisées en terme de capacité de croissance et rendement en particules virales, avant d'envisager leur mise en oeuvre dans un processus industriel. De plus, une lignée complémentant l'ensemble des fonctions adénovirales adaptée à la propagation des vecteurs minimaux n'est à l'heure actuelle pas encore disponible.

Une autre alternative repose sur l'emploi d'un élément viral supplémentaire, désigné "virus auxiliaire" pour complémenter au moins en partie les fonctions défectives d'un vecteur adénoviral recombinant. Les virus auxiliaires de l'art antérieur consistent en un génome adénoviral, éventuellement délété d'une région essentielle pour laquelle le vecteur recombinant ne nécessite pas de complémentation. A titre d'exemple, la co-transfection dans la lignée 293 d'un virus auxiliaire E1⁻ et d'un vecteur adénoviral recombinant E1⁻ E4⁻ conduit à la formation de particules virales de vecteur recombinant. La fonction E1 est fournie par la lignée 293 et la fonction E4 par le virus auxiliaire.

Cependant, un inconvénient majeur de cette méthode est que les cellules produisent une population mixte de particules virales, les unes comportant le vecteur recombinant et les autres le vecteur auxiliaire. En pratique, les préparations contiennent majoritairement des particules virales auxiliaires, celles-ci présentant un avantage sélectif, de sorte que la contamination peut atteindre et même dépasser 90%. La présence du virus auxiliaire n'est pas souhaitable dans le cadre d'une thérapie appliquée à l'homme et, de ce fait, nécessite la mise en oeuvre de techniques physiques de séparation, telles que l'ultracentrifugation.

La présente invention se propose d'exploiter les propriétés de croissance respectives des adénovirus humains et animaux. On a maintenant mis en évidence l'incapacité des adénovirus bovins BAV3 à se propager dans une lignée humaine alors que l'Ad5 peut être propagé dans des cellules bovines. En effet, l'infection d'adénovirus BAV3 seuls ou en présence d'Ad5 dans la lignée humaine 293 ne conduit pas à la formation de particules virales infectieuses BAV3. Par contre des virions Ad5 sont obtenus par infection d'une lignée bovine. En outre, il n'y a pas d'expression des protéines virales BAV3 dans les cellules humaines.

Sur la base de ces observations, la présente invention propose notamment un système d'encapsidation se déroulant en deux étapes et mettant en oeuvre des adénovirus chimères entre l'Ad5 et BAV3. On a maintenant construit (i) un vecteur auxiliaire dérivé d'un génome Ad5 dans lequel la région d'encapsidation native est remplacée celle de l'adénovirus bovin BAV3 et (ii) un vecteur adénoviral défectif recombinant dérivant d'un Ad5 et comprenant deux régions d'encapsidation, la première d'origine Ad5 (autologue) et la seconde d'origine BAV3 (hétérologue). La transfection des deux vecteurs dans une lignée cellulaire bovine infectée par un adénovirus BAV3 conduit à l'amplification des trois génomes viraux et à la production de particules virales des trois types. Au cours de cette première étape d'amplification, le génome BAV3 fournit les facteurs agissant *en trans* permettant l'encapsidation des vecteurs recombinant et auxiliaire et ce dernier complémente au moins en partie les fonctions défectives du vecteur recombinant. Le mélange des trois types de virus est récupéré des cellules bovines et utilisé pour infecter des cellules humaines 293. Le génome BAV3 et le vecteur auxiliaire possédant uniquement une région d'encapsidation dérivée de BAV3 ne peuvent pas se propager dans la lignée humaine même en présence d'Ad5 du fait de l'absence des facteurs d'encapsidation reconnaissant les séquences BAV3, ce qui exclut la formation de particules virales correspondantes. Neanmoins, le vecteur auxiliaire peut produire *en trans* les facteurs nécessaires à l'encapsidation du vecteur recombinant médiée par le signal d'encapsidation d'origine Ad5 et complémenter, en association avec les cellules 293, les fonctions précoces et tardives défectives, dans le but de générer de façon majoritaire des virions contenant le vecteur recombinant. La présente invention répond à des objectifs de sécurité en réduisant considérablement la contamination des préparations adénovirales par les vecteurs auxiliaires et évite ainsi la mise en oeuvre de techniques de séparation longues, coûteuses et d'efficacité variable.

C'est pourquoi la présente invention a pour objet un vecteur adénoviral dérivant d'un génome adénoviral caractérisé en ce qu'il comprend une région essentielle à l'encapsidation d'origine adénovirale hétérologue par rapport au génome adénoviral dont il dérive.

Au sens de la présente invention, un vecteur adénoviral est obtenu à partir d'un adénovirus parental dont le génome est modifié. Une modification minimale est l'insertion d'une région essentielle à l'encapsidation d'une origine différente (hétérologue). Bien entendu, d'autres modifications peuvent également être envisagées. Celles-ci peuvent être diverses (délétion, addition, substitution d'un ou plusieurs nucléotides) et localisées au sein des régions codantes du génome adénoviral ou en dehors de celles-ci (régions impliquées dans l'expression des gènes viraux, dans l'encapsidation....) et touchées aussi bien les régions précoces que tardives. A cet égard, un vecteur adénoviral particulièrement adapté à la présente invention est défectif, c'est à dire incapable de se propager de façon autonome dans une cellule hôte en l'absence de complémentation. Il peut être défectif pour un ou plusieurs gènes viraux essentiels à la réplication. Ces gènes peuvent être délétés (en totalité ou en partie), rendus non fonctionnels (par exemple par mutation) ou substitués par d'autres séquences (notamment par un gène d'intérêt dont l'expression est recherchée dans une cellule ou un organisme hôte).

Le vecteur adénoviral de la présente invention peut dériver d'un adénovirus humain ou animal et d'un sérotype quelconque. Les adénovirus humains du sous groupe C et notamment les adénovirus 2 (Ad2) et 5 (Ad5) conviennent tout particulièrement à la mise en oeuvre de l'invention. Parmi les adénovirus animaux utilisables dans le cadre de la présente invention, on peut citer les adénovirus canins, aviaires, bovins, murins, ovins porcins, simiens...etc. A titre illustratif, on peut employer les adénovirus murins Mavl (Beard et al., 1990, Virology *175*, 81-90), canins CAV-1 ou CAV-2 (Spibey et Cavanagh, J. Gen. Virol., 1989, *70*, 165-172; Linné, 1992, Virus Research *23*, 119-133 ; Shibata et al., 1989, Virol. *172*, 460-467; Jouvenne et al., Gene, 1987, *60*, 21-28), aviaires DAV (Zakharchuk et al., Arch. Virol., 1993, *128*, 171-176) ou encore bovins BAV3 (Mittal et al., J. Gen. Virol., 1995, *76*, 93-102). D'une manière générale, les adénovirus précités sont disponibles dans les collections et notamment à l'ATCC et ont fait l'objet de nombreuses études publiées dans l'art antérieur. Concernant l'adénovirus 5 (Ad5), il convient de noter que la séquence complète de son génome est disponible auprès de GenBank sous le numéro d'accession M73260. Cette séquence est intégralement incorporée par référence dans la présente demande.

Aux fins de la présente invention, par "région essentielle à l'encapsidation", on entend une région agissant *en cis* pour assurer, en collàboration avec des facteurs protéiques notamment viraux, l'encapsidation d'un génome de vecteur viral dans une capside virale. De telles régions consistent notamment, dans le cas du génome adénoviral, en les ITR 5' et 3', et la région d'encapsidation. Ces termes sont bien connus dans le domaine de l'art considéré.

La caractéristique du vecteur adenoviral selon l'invention est qu'il porte une région essentielle à l'encapsidation hétérologue c'est à dire d'une origine différente par rapport à l'adénovirus parental. De manière préférée, selon la présente invention, ladite région essentielle à l'encapsidation hétérologue consiste en la séquence d'encapsidation et éventuellement en l'un au moins des ITR 5' et 3'.

Selon l'origine de l'adénovirus, les régions essentielles à l'encapsidation peuvent varier quelque peu. Néanmoins, elles peuvent être identifiées sur la base des données de séquence disponibles ou par analogie avec les adénovirus humains. Les ITR sont naturellement localisés aux extrémités 5' et 3' du génome adénoviral et sont impliqués dans les étapes de réplication et d'encapsidation dudit génome. Généralement, les ITR comportent entre 100 et 200 paires de bases. De nombreuses séquences d'ITR sont proposées par la littérature, citons à titre d'exemple Hearing et al., 1987, J. Virol., 61, 2555-2558 pour l'Ad5 ou WO 95/16048 pour BAV3. La région d'encapsidation (notée Ψ) est localisée à la suite de l'ITR 5' du génome adénoviral et comporte des motifs redondants qui participent à l'encapsidation. Par exemple, celle de l'Ad5 comporte 7 motifs désignés AI à AVII de séquence consensus 5' A/T AN A/T TTTG 3' (où N représente un nucléotide quelconque) et situés aux positions 241-248, 262-269, 304-311, 314-321 et 339-346 du génome viral (Grable et Hearing, 1990, J. Virol. *64*, 2047-2056; Schmid et Hearing, 1998, J. Virol. *72*, 6339-6347). Les régions d'encapsidation de divers adénovirus sont décrites dans la littérature (voir par exemple Hammarskjold et Winberg, 1980, Cell *20*, 787-795 pour l'Adl6 ; Hearing et al., 1987, J. Virol. *61*, 2555-2558 pour l'Ad5 ; Robinson et Tibbets, 1984, Virology *137*, 276-286 pour l'Ad3 ; Shibata et al., 1989, Virology 172, 460-467 pour CAV2 ; WO95/16048 pour BAV3). A titre purement illustratif, on indique que la région d'encapsidation d'Ad5 s'étend au moins des nucléotides (nt) 240 à 350. Quant à celle de BAV3, elle est comprise au sein d'un fragment de 0,3 kb entre les positions environ 185 à environ 514. Néanmoins, les limites de la région d'encapsidation peuvent varier et des régions plus courtes ou plus longues conviennent également. L'homme du métier est capable d'isoler un fragment de l'extrémité 5' d'un génome adénoviral, de l'insérer dans un vecteur adéquat et de vérifier ses capacités d'encapsidation dans une lignée appropriée, par exemple en déterminant le titre viral ou l'expression d'un gène reporter.

Les séquences portant la région essentielle à l'encapsidation hétérologue peuvent être isolées d'un génome viral par des moyens conventionels (digestion par enzyme de restriction, PCR ...) ou produites par synthèse chimique. Eventuellement, dans le cadre de la présente invention, elles peuvent comprendre des mutations (délétion, substitution et/ou addition d'un ou plusieurs nucléotides) par rapport aux séquences natives. Il est également possible d'inclure d'autres séquences exogènes (sites de restriction... etc). Elles peuvent être insérée dans le vecteur adénoviral selon l'invention *en sus* de la région autologue ou en remplacement de cette dernière. L'insertion peut avoir lieu en 5' ou en 3' de la région autologue, à sa place ou à un endroit différent (par exemple à l'extrémité 3' juste avant l'ITR 3').

Avantageusement, le vecteur adénoviral selon l'invention est originaire d'un adénovirus d'origine humaine et la région essentielle à l'encapsidation hétérologue d'un adénovirus d'origine animale. A cet égard, un vecteur convenant tout particulièrement à la présente invention dérive d'un adénovirus humain du sous groupe C et, notamment d'un adénovirus 2 (Ad2) ou 5 (Ad5). Quant à la région essentielle à l'encapsidation hétérologue, elle dérive de préférence d'un adénovirus animal sélectionné parmi ceux précédemment cités.

Selon un mode de réalisation tout à fait préféré, le vecteur adénoviral selon l'invention dérive d'un Ad5 et la région essentielle à l'encapsidation hétérologue d'un adénovirus bovin, notamment d'un BAV3.

On indique que les modes de réalisation précités sont préférés mais que d'autres combinaisons peuvent être mises en oeuvre dans le cadre de la présente invention. On peut par exemple envisager un vecteur adénoviral dérivant d'un Ad5 et comprenant une région essentielle à l'encapsidation hétérologue issue d'un autre adénovirus humain de sérotype différent (Ad3, Ad7 ...etc). Alternativement, le squelette adénoviral peut être d'origine animale et la région essentielle à l'encapsidation issue d'un adénovirus humain.

Comme indiqué auparavant, le vecteur adénoviral selon l'invention est de préférence défectif au moins pour la fonction E1. Une telle déficience peut être obtenue par délétion totale ou partielle de la région correspondante. De nombreux vecteurs E1⁻ sont décrits dans l'art antérieur et peuvent être utilisés dans le cadre de la présente invention. En outre, il peut comprendre des mutations /délétions supplémentaires affectant un ou plusieurs autres gènes viraux, notamment au sein des régions E2, E4 et/ou L1-L5. Toutes les combinaisons peuvent être envisagées (E1⁻ E2⁻, E1⁻ E4⁻, E1⁻ E2⁻ E4⁻ ...). De tels vecteurs sont notamment décrits dans la demande internationale WO94/28152. Pour illustrer ces modes de réalisation, on peut citer la mutation thermosensible affectant le gène DBP (pour DNA Binding Protein en anglais) de la région E2A (Ensinger et al., 1972, J. Virol. *10*, 328-339). Une délétion partielle de la région E4 à l'exception des séquences codant pour les cadres de lecture ouverts (ORF) 6 et 7 est également envisageable (Ketner et al., 1989, Nucleic Acids Res. *17*, 3037-3048). Une autre possibilité est la délétion totale de l'unité transcriptionnelle E4. Par ailleurs, le vecteur adénoviral selon l'invention peut être dépourvu de tout ou partie de la région non essentielle E3. Selon cette alternative, il peut être intéressant de conserver néanmoins les séquences E3 codant pour les polypeptides permettant l'échappement au système immunitaire de l'hôte, notamment la glycoprotéine gp19k (Gooding et al., 1990, Critical Review of Immunology *10*, 53-71). Dans certaines applications (vecteur recombinant), la non-fonctionnalité de l'ensemble des gènes viraux est préférée.

Selon une première variante, le vecteur adénoviral selon l'invention peut être employé à titre de vecteur viral auxiliaire pour complémenter tout ou partie des fonctions défectives d'un vecteur adénoviral recombinant. Selon un mode de réalisation avantageux, il est défectif au moins pour la fonction E1. Eventuellement, il peut être défectif pour des fonctions supplémentaires telles que E2. Un homme du métier est apte à définir les déficiences requises en fonction du vecteur recombinant que l'on cherche à complémenter et de la lignée cellulaire choisie. On indique que la fonction E4 peut être assurée par l'ORF 6 et 7 uniquement. La présence de tout ou partie de la région E3 est facultative. Selon un mode de réalisation particulier, il comprend les séquences ITR 5' et 3' et les séquences codant pour les fonctions E2, E4 et/ou L1-L5 dérivant d'un adénovirus humain, notamment d'un Ad5 et une région d'encapsidation hétérologue dérivant d'un adénovirus bovin, notamment d'un BAV3. Selon un autre mode de réalisation, le vecteur viral auxiliaire comprend les séquences codant pour les fonctions E2, E4 et/ou L1-L5 dérivant d'un adénovirus humain, notamment d'un Ad5 et des séquences ITR 5', ITR 3' et région d'encapsidation hétérologues, dérivant d'un adénovirus bovin, notamment d'un BAV3.

On obtient un vecteur adénoviral auxiliaire selon l'invention par insertion dans un génome adénoviral tel que défini ci-avant d'au moins une région essentielle à l'encapsidation hétérologue. Une manière préférée de procéder est de remplacer la région essentielle à l'encapsidation autologue par l'hétérologue. L'homme du métier est apte à réaliser une telle construction en appliquant les techniques classiques de biologie moléculaire.

Selon une seconde variante, le vecteur adénoviral selon l'invention est un vecteur adénoviral recombinant et comprend au moins un gène d'intérêt placé sous le contrôle des éléments nécessaires à son expression dans une cellule ou un organisme hôte.

Le gène d'intérêt en usage dans la présente invention, peut être issu d'un organisme eucaryote, d'un procaryote d'un parasite ou d'un virus autre qu'un adénovirus. Il peut être isolé par toute technique conventionnelle dans le domaine de l'art, par exemple par clonage, PCR ou synthèse chimique. Il peut être de type génomique (comportant tout ou partie de l'ensemble des introns), de type ADN complémentaire (ADNc, dépourvu d'intron) ou de type mixte (minigène). Par ailleurs, il peut coder pour un ARN antisens et/ou un ARN messager (ARNm) qui sera ensuite traduit en polypeptide d'intérêt celui-ci pouvant être (i) intracellulaire, (ii) incorporé dans la membrane de la cellule hôte ou (iii) secrété. Il peut s'agir d'un polypeptide tel que trouvé dans la nature (natif), d'une portion de celui-ci (tronqué), d'un mutant présentant notamment des propriétés biologiques améliorées ou modifiées ou encore d'un polypeptide chimère provenant de la fusion de séquences d'origines diverses.

Dans le cadre de la présente invention, il peut être avantageux d'utiliser un gène d'intérêt codant pour une cytokine (interféron α, β ou γ, interleukine (IL), notamment l'IL-2, l'IL-6, l'IL-10 ou encore l'IL-12, un facteur nécrosant des tumeurs (TNF), un facteur stimulateur de colonies (GM-CSF, C-CSF, M-CSF...), un récepteur cellulaire (notamment reconnu par le virus HIV), un ligand de récepteur, un facteur de coagulation, un facteur de croissance (FGF pour Fibroblast Growth Factor, VEGF pour Vascular Endothelial Growth Factor), une enzyme (uréase, rénine, thrombine, métalloprotéinase, nitric oxide synthétase NOS, SOD, catalase...), un inhibiteur d'enzyme (α1-antitrypsine, antithrombine III, inhibiteur de protéase virale, PAI-1 pour plasminogen activator inhibitor), un antigène du complexe majeur d'histocompatibilité de classe I ou II ou un polypeptide agissant sur l'expression des gènes correspondants, un polypeptide capable d'inhiber une infection virale, bactérienne ou parasitaire ou son développement, un polypeptide agissant positivement ou négativement sur l'apoptose (Bax, Bcl2, BclX...), un agent cytostatique (p21, p 16, Rb), une apolipoprotéine (ApoAI, ApoAIV, ApoE...), un inhibiteur d'angiogénèse (angiostatine, endostatine...), un marqueur (β-galactosidase, luciférase....) ou tout autre gène d'intérêt ayant un effet thérapeutique pour l'affection ciblée. Plus précisemment, dans le but de traiter un dysfonctionnement héréditaire, on utilisera une copie fonctionnelle du gène défectueux, par exemple un gène codant pour le facteur VIII ou IX dans le cadre de l'hémophilie A ou B, la dystrophine dans le cadre des myopathies de Duchenne et Becker, l'insuline dans le cadre du diabète, la protéine CFTR (Cystic Fibrosis Transmembrane Conductance Regulator) dans le cadre de la mucoviscidose. S'agissant d'inhiber l'initiation ou la progression de tumeurs ou cancers, on mettra de préférence en oeuvre un gène d'intérêt codant pour un ARN anti-sens, un ribozyme, un produit cytotoxique (thymidine kinase de virus simplex de l'herpès 1 (TK-HSV-1), ricine, toxine cholérique, diphtérique, produit des gènes de levure *FCY1* et *FUR1* codant pour l'uracyle phosphoribosyl transférase et la cytosine désaminase.....), un anticorps, un inhibiteur de la division cellulaire ou des signaux de transduction, un produit d'expression d'un gène suppresseur de tumeur (p53, Rb, p73....), un polypeptide stimulateur du système immunitaire, un antigène associé à une tumeur (MUC-1, BRCA-1, antigènes précoces ou tardifs (E6, E7, L1, L2...) d'un virus à papillome HPV....), éventuellement en combinaison avec un gène de cytokine. Enfin, dans le cadre d'une thérapie anti-HIV, on peut avoir recours à un gène codant pour un polypeptide immunoprotecteur, un épitope antigénique, un anticorps (2F5; Buchacher et al., 1992, Vaccines *92*, 191-195), le domaine extracellulaire du récepteur CD4 (sCD4 ; Traunecker et al., 1988, Nature *331*, 84-86) une immunoadhésine (par exemple un hybride CD4-immunoglobuline IgG ; Capon et al., 1989, Nature *337*, 525-531 ; Byrn et al., 1990, Nature *344*, 667-670), une immunotoxine (par exemple fusion de l'anticorps 2F5 ou de l'immunoadhésine CD4-2F5 à l'angiogénine ; Kurachi et al., 1985, Biochemistry *24*, 5494-5499), un variant trans-dominant (EP 0614980, WO95/16780), un produit cytotoxique tel que l'un de ceux mentionné ci-dessus ou encore un IFNα ou β.

Par ailleurs, un des gènes d'intérêt peut également être un gène de sélection permettant de sélectionner ou identifier les cellules transfectées ou transduites. On peut citer les gènes *néo* (codant pour la néomycine phosphotransférase) conférant une résistance à l'antibiotique G418, *dhfr* (Dihydrofolate Réductase), CAT (Chloramphenicol Acetyl transférase), *pac* (Puromycine Acétyl-Transferase) ou encore *gpt* (Xanthine Guanine Phosphoribosyl Transferase). D'une manière générale, les gènes de sélection sont connus de l'homme de l'art.

La locution "éléments nécessaires à l'expression" désigne les éléments génétiques permettant la transcription d'un gène d'intérêt en ARN et la traduction d'un ARNm en polypeptide. Parmi ceux-ci, le promoteur revêt une importance particulière. Il peut être isolé d'un gène quelconque d'origine eucaryote ou même virale et peut être constitutif ou régulable. Alternativement, il peut s'agir du promoteur naturel du gène en question. Par ailleurs, il peut être modifié de manière à améliorer l'activité promotrice, supprimer une région inhibitrice de la transcription, rendre un promoteur constitutif régulable ou *vice versa*, introduire un site de restriction.... On peut mentionner, à titre d'exemples, les promoteurs eucaryotes des gènes PGK (Phospho Glycérate Kinase), MT (metallothioneine ; Mc Ivor et al., 1987, Mol. Cell Biol. *7*, 838-848), α-1 antitrypsine, CFTR, surfactant, immunoglobuline, β-actine (Tabin et al., 1982, Mol. Cell Biol. *2*, 426-436), SRα (Takebe et al., 1988, Mol. Cell. Biol. *8*, 466-472), le promoteur précoce du virus SV40 (Simian Virus), le LTR du RSV (Rous Sarcoma Virus), le promoteur TK-HSV-1, le promoteur précoce du virus CMV (Cytomegalovirus) et les promoteurs adénoviraux E1A et MLP. Il peut également s'agir d'un promoteur stimulant l'expression dans une cellule tumorale ou cancéreuse. On peut citer notamment les promoteurs des gènes MUC-1 surexprimé dans les cancers du sein et de la prostate (Chen et al., 1995, J. Clin. Invest. *96*, 2775-2782), CEA (pour carcinoma embryonic antigen) surexprimé dans les cancers du colon (Schrewe et al., 1990, Mol. Cell. Biol. *10*, 2738-2748), tyrosinose surexprimé dans les mélanomes (Vile et al., 1993, Cancer Res. *53*, 3860-3864), ERB-2 surexprimé dans les cancers du sein et du pancréas (Harris et al., 1994, Gene Therapy *1*, 170-175) et α-fétoprotéine surexprimée dans les cancers du foie (Kanai et al., 1997, Cancer Res. *57*, 461-465). Le promoteur précoce du Cytomégalovirus (CMV) est tout particulièrement préféré.

Les éléments nécessaires à l'expression peuvent, en outre, inclure des éléments additionnels améliorant l'expression du gène d'intérêt ou son maintien dans la cellule hôte. On peut citer notamment les séquences introniques, séquences signal de sécrétion, séquences de localisation nucléaire, sites internes de réinitiation de la traduction de type IRES, séquences poly A de terminaison de la transcription, leaders tripartites et origines de réplication. Ces éléments sont connus de l'homme de l'art.

Lorsque le vecteur adénoviral recombinant selon l'invention comporte plusieurs gènes d'intérêt, ceux-ci peuvent être placés sous le contrôle des mêmes éléments génétiques (cassette polycistronique utilisant un site interne d'initiation de la traduction de type IRES pour réinitier la traduction du second cistron) ou d'éléments indépendants.

Un mode de réalisation particulièrement avantageux consiste en un vecteur adénoviral recombinant comprenant une seconde région essentielle à l'encapsidation autologue par rapport au génome adénoviral dont il dérive. En d'autres termes, il porte deux régions d'encapsidation l'une autologue et l'autre hétérologue. Leur position dans le vecteur adénoviral peut être quelconque. Elles peuvent être notamment placées à l'une des extrémités ou séparées à chacune des extrémités dudit vecteur. Avantageusement, la région hétérologue est placée en 3' de la région autologue.

Dans le cadre de la présente invention, un vecteur adénoviral recombinant selon l'invention est défectif pour la fonction E1 et l'une au moins des fonctions E2, E4 et/ou L1-L5. Un exemple préféré est fourni par un vecteur défectif pour l'ensemble des fonctions adénovirales (vecteur minimum) qui comprend outre le(s) gène(s) d'intérêt au moins les ITRs 5' et 3' et une région essentielle à l'encapsidation autologue dérivant d'un adénovirus humain, notamment d'un Ad5 et une région essentielle à l'encapsidation hétérologue dérivant d'un adénovirus bovin, notamment d'un BAV3.

Il est à la portée de l'homme du métier de générer un vecteur adénoviral recombinant selon l'invention par les techniques de biologie moléculaire. Il saura bien évidemment adapter la technologie en fonction des données spécifiques (type de vecteur, gène d'intérêt...).

La présente invention concerne également l'utilisation d'un vecteur adénoviral auxiliaire selon l'invention pour complémenter tout ou partie des fonctions défectives d'un vecteur adénoviral recombinant défectif pour la réplication, notamment d'un vecteur adénoviral recombinant selon l'invention, lesdits vecteurs auxiliaire et recombinant dérivant tous deux d'un même adénovirus et, en particulier d'un Ad5 et lesdites régions essentielles à l'encapsidation hétérologues portées par le vecteur auxiliaire et, éventuellement, le vecteur recombinant dérivant du même adénovirus différent du précédent et, en particulier d'un BAV3.

La présente invention concerne également une composition comprenant :
(a) un vecteur adénoviral auxiliaire selon l'invention,
(b) un vecteur adénoviral recombinant défectif pour la réplication, notamment d'un vecteur adénoviral recombinant selon l'invention, et
(c) de manière optionnelle, un génome adénoviral dérivant d'un adénovirus animal de la même origine que les régions essentielles à l'encapsidation hétérologues portées par les vecteurs auxiliaire et recombinant a) et éventuellement b).

La présente invention concerne également un génome adénoviral dérivant d'un adénovirus animal, notamment d'un adénovirus bovin et, en particulier, d'un BAV3 caractérisé en ce qu'il présente une capacité d'encapsidation atténuée par rapport à l'adénovirus dont il dérive. L'atténuation a pour but de réduire la propagation du génome adénoviral au profit d'un génome portant une région native (vecteur adénoviral auxiliaire et recombinant selon l'invention). Elle peut être obtenue par délétion partielle de la région d'encapsidation ou par mutation d'un ou plusieurs motifs contrôlant le processus d'encapsidation. Un exemple d'atténuation est fourni dans la demande internationale WO94/28152 et dans Imler et al. (1995, Human Gene Therapy *6*, 711-721). L'homme de l'art connaît les techniques qui permettent de vérifier l'atténuation, par exemple en déterminant le titre viral (Graham et Prevec, 1991, supra) ou l'expression d'un gène reporter par rapport à un virus équivalent portant une région essentielle à l'encapsidation native. Une région essentielle à l'encapsidation atténuée présente une efficacité d'encapsidation réduite d'un facteur 2 à 1000, avantageusement 3 à 100 et, de manière préférée, 5 à 50.

Le génome adénoviral animal selon l'invention peut être compétent pour la replication ou comprendre des modifications touchant un ou plusieurs gènes viraux, telles que celles citées précédemment. En particulier, la délétion totale ou partielle de la région E1 dudit génome peut être avantageuse dans le cadre de la présente invention. On indique que le génome et/ou les vecteurs peuvent être sous forme d'ADN ou de virus.

La présente invention concerne également un procédé pour préparer une préparation virale comprenant un vecteur adénoviral recombinant défectif pour la réplication, notamment d'un vecteur adénoviral recombinant selon l'invention, selon lequel :
(a) on introduit dans une première lignée cellulaire
   (i) un vecteur adénoviral auxiliaire selon l'invention,
   (ii) un genome adénoviral dérivant d'un adénovirus animal, et
   (iii) ledit vecteur adénoviral recombinant
   ledit génome adénoviral (ii) étant de la même origine que les régions essentielles à l'encapsidation hétérologues portées par les vecteurs (i) et éventuellement (iii) et ledit génome adénoviral (ii) et les vecteurs (i) et (iii) étant capables de se répliquer dans ladite première lignée cellulaire,
(b) on cultive ladite première lignée cellulaire dans des conditions appropriées pour permettre la production de particules virales comportant les vecteurs (i) et (iii) et le génome adénoviral (ii),
(c) on récupère lesdites particules virales obtenues à l'étape b) dans la culture cellulaire,
(d) on infecte une seconde lignée cellulaire avec lesdites particules virales récupérées à l'étape c), ledit vecteur auxiliaire (i) et ledit génome adénoviral (ii) ayant une capacité d'encapsidation et/ou de réplication nulle ou réduite dans ladite seconde lignée cellulaire,
(e) on cultive ladite seconde lignée cellulaire dans des conditions appropriées pour permettre l'encapsidation dudit vecteur adénoviral recombinant (iii) et produire ladite préparation virale, et
(f) on récupère ladite préparation virale obtenue à l'étape e) dans la culture cellulaire.

Aux fins de la présente invention, les vecteurs et génomes adénoviraux peuvent être introduits par tous les moyens de l'art dans la première lignée cellulaire, notamment par transfection et/ou infection. Il est possible de transfecter les vecteurs dans la lignée qui est infectée par des particules de génome adénoviral animal (préalablement, postérieurement ou de manière concomitante à la transfection). Les virus contenant les différents éléments (i) (ii) ou (iii) peuvent être constitués selon les techniques de l'art. Par ailleurs, ledit vecteur adénoviral recombinant défectif pour la réplication peut consister en un vecteur tel que décrit dans WO 94/28152, WO 94/08026, WO 93/19191 ou WO 94/12649.

Par ailleurs, le génome (ii) peut être sauvage, selon l'invention (atténué) et/ou comprendre une ou plusieurs modifications affectant la fonctionnalité d'un ou de plusieurs gènes viraux.

Selon un mode de mise en oeuvre avantageux, la première cellule comprend
(i) un vecteur adénoviral auxiliaire défectif au moins pour la fonction E1, dérivant d'un adénovirus humain (notamment d'un Ad5) et portant une région essentielle à l'encapsidation hétérologue dérivant d'un adénovirus bovin (notamment d'un BAV3),
(ii) un génome adénoviral dérivant d'un adénovirus bovin (notamment d'un BAV3), éventuellement selon l'invention, et
(iii) vecteur adénoviral recombinant défectif pour la fonction E1 et au moins l'une des fonctions E2, E4 et/ou L1-L5 et portant une seconde région d'encapsidation autologue telle que définie ci-avant, dérivant d'un adénovirus humain (notamment d'un Ad5) et portant une région essentielle à l'encapsidation hétérologue dérivant d'un adénovirus bovin (notamment d'un BAV3),
ladite première lignée cellulaire étant d'origine bovine.

Selon un mode de réalisation tout à fait avantageux, le vecteur adénoviral auxiliaire défectif dont il est question en (i) des procédé décrits ci-dessus comprend une région essentielle à l'encapsidation hétérologue dérivant d'un adénovirus bovin (notamment d'un BAV3) qui comporte les ITR 5' et 3', et la région d'encapsidation.

De manière alternative on peut avoir recours à un génome adénoviral (ii) défectif pour la fonction E1. S'agissant de la variante préférée, un génome BAV3 défectif pour la fonction E1 est décrit dans la demande internationale WO95/16048. Dans ce cas, on aura recours à une première lignée cellulaire capable de complementer la fonction E1 dudit génome adénoviral d'origine animale. On emploiera de préférence une lignée de la même origine animale que ledit génome adénoviral (ii). Il peut s'agir d'une lignée établie ou d'une lignée primaire.

Le terme cellule de complémentation est classique dans le domaine de l'art. Dans le cadre de la présente invention, il se réfère à une cellule eucaryote capable de fournir *en trans* au moins une partie des fonctions défectueuses d'un vecteur ou génome adénoviral selon l'invention. D'une manière générale, une cellule de complémentation d'une fonction adénovirale peut être obtenue par transfection des gènes viraux correspondant dans une lignée cellulaire appropriée. Tous les moyens standards pour introduire un ADN dans une cellule peuvent être employés (transfection au phosphate de calcium, electroporation, microinjection, lipofection, fusion de protoplastes....). Par ailleurs, les gènes viraux sont portés par des vecteurs conventionnels (vecteurs synthétiques, viraux, plasmides...) et placés sous le contrôle d'éléments permettant leur expression constitutive ou régulée dans ladite cellule de complémentation. Les lignées de complémentation adaptées aux vecteurs adénoviraux sont connues de l'homme de l'art (voir par exemple la demande internationale WO94/28152, WO97/04119 et Graham et al., J. Gen. Virol., 1977, *36*: 59-72).

Une lignée bovine convenant particulièrement dérive d'une lignée MDBK (ATCC CCL-22 ou CRL-6071) ou de cellules primaires, notamment de rétine ou de rein foetal et comprend les séquences codant pour la région E1 d'un adénovirus bovin et, en particulier d'un BAV3 placées sous le contrôle des éléments nécessaires à leur expression dans ladite lignée.

Selon un mode de réalisation préféré, la seconde lignée cellulaire est une cellule de complémentation de la fonction E1 d'un adénovirus humain, notamment d'un Ad5. On aura de préférence recours à la lignée 293. Mais d'autres lignées telles que celles décrites dans la demande WO94/28152 peuvent également être employées.

Les particules virales de l'étape c) et la préparation virale peuvent être récupérées du surnageant de culture mais également des cellules. Une des méthodes couramment employée consiste à lyser les cellules par des cycles consécutifs de congélation/décongélation pour recueillir les virions dans le surnageant de lyse. Ceux-ci peuvent ensuite être amplifiés et purifiés selon les techniques de l'art (procédé chromatographique, ultracentrifugation notamment à travers un gradient de chlorure de césium....).

La présente invention concerne également la préparation virale obtenue selon le procédé selon l'invention. Selon un mode de réalisation avantageux, elle comporte au moins 30% de particules virales infectieuses contenant le vecteur adénoviral recombinant selon l'invention. Avantageusement, elle comporte au moins 50%, de préférence, au moins 70% et, de manière tout à fait préférée, au moins 80% desdites particules.

La présente invention concerne également une cellule hôte comprenant un vecteur adénoviral selon l'invention ou infectée par une préparation virale selon l'invention. Une cellule de mammifère et notamment humaine convient tout particulièrement. Elle peut comprendre ledit vecteur sous forme intégrée dans le génome ou non (épisome). Il peut s'agir d'une cellule primaire ou tumorale d'une origine hématopoïétique (cellule souche totipotente, leucocyte, lymphocyte, monocyte ou macrophage...), musculaire (cellule satellite, myocyte, myoblaste...), cardiaque, pulmonaire, trachéale, hépatique, épithéliale ou fibroblaste.

La présente invention concerne également une cellule comprenant :
(i) un vecteur adénoviral auxiliaire selon l'invention,
(ii) un génome adénoviral dérivant d'un adénovirus animal, et
(iii) un vecteur adénoviral recombinant défectif pour la réplication, notamment d'un vecteur adénoviral recombinant selon l'invention.

Ladite cellule est de préférence une cellule de complémentation d'une fonction adénovirale et , en particulier, de la fonction E1 d'un adénovirus animal ou humain. Elle a les caractéristiques définies ci-avant.

La présente invention concerne également une composition pharmaceutique comprenant à titre d'agent thérapeutique ou prophylactique, un vecteur adénoviral, une préparation virale ou une cellule hôte selon l'invention en association avec un support acceptable d'un point de vue pharmaceutique. La composition selon l'invention est plus particulièrement destinée au traitement préventif ou curatif de maladies par thérapie génique et s'adresse aussi bien aux maladies génétiques (hémophilie, diabète, mucoviscidose, myopathie de Duchenne ou de Becker, maladies auto immunes) qu'acquises (cancers, tumeurs, maladies cardiovasculaires, maladies d'origine infectieuse comme l'hépatite B ou C, le SIDA....).

Une composition pharmaceutique selon l'invention peut être fabriquée de manière conventionnelle en vue d'une administration par voie locale, parentérale ou digestive. En particulier, on associe une quantité thérapeutiquement efficace de l'agent thérapeutique ou prophylactique à un support acceptable d'un point de vue pharmaceutique. Les voies d'administration envisageables sont multiples. On peut citer par exemple la voie intragastrique, sous-cutanée, intracardiaque, intramusculaire, intraveineuse, intraartérielle, intrapéritonéale, intratumorale, intranasale, intrapulmonaire ou intratrachéale. Pour ces trois derniers modes de réalisation, une administration par aérosol ou instillation est avantageuse. L'administration peut avoir lieu en dose unique ou répétée une ou plusieurs fois après un certain délai d'intervalle. La voie d'administration et le dosage appropriés varient en fonction de divers paramètres, par exemple, de l'individu ou de la maladie à traiter ou encore du ou des gène(s) d'intérêt à transférer. La préparation virale selon l'invention peuvent être formulées sous forme de doses comprises entre 10⁴ et 10¹⁴ ufp (unités formant des plages), avantageusement 10⁵ et 10¹³ ufp et, de préférence, 10⁶ et 10¹² ufp. Pour ce qui est du vecteur adénoviral recombinant selon l'invention, des doses comprenant de 0,01 à 100 mg d'ADN, de préférence 0,05 à 10 mg et, de manière tout à fait préférée, 0,5 à 5 mg sont envisageables. La formulation peut également inclure un diluant, un adjuvant ou un excipient acceptable d'un point de vue pharmaceutique. Elle peut être présentée sous forme liquide ou sèche (lyophylisat ... etc).

Le vecteur ou la préparation virale selon l'invention peut être éventuellement associé à une ou plusieurs substances améliorant l'efficacité transfectionnelle et/ou la stabilité. Ces substances sont largement documentées dans la littérature accessible à l'homme de l'art (voir par exemple Felgner et al., 1987, Proc. West. Pharmacol. Soc. *32*, 115-121 ; Hodgson et Solaiman, 1996, Nature Biotechnology *14*, 339-342; Remy et al., 1994, Bioconjugate Chemistry *5*, 647-654). A titre illustratif mais non limitatif, il peut s'agir de polymères, de lipides notamment cationiques, de liposomes, de protéines nucléaires ou encore de Lipides neutres. Ces substances peuvent être utilisées seules ou en combinaison.

Enfin, la présente invention est relative à l'utilisation d'un vecteur adénoviral, d'une préparation virale ou d'une cellule hôte selon l'invention pour le transfert et l'expression d'un gène d'intérêt dans une cellule ou un organisme hôte. Une utilisation préférée consiste en le traitement du corps humain ou animal par thérapie génique ou immunothérapie. Selon une première possibilité, le médicament peut être administré directement *in vivo* (par exemple par injection intraveineuse, dans une tumeur accessible, dans les poumons par aérosol...). On peut également adopter l'approche *ex vivo* qui consiste à prélever des cellules du patient (cellules souches de la moëlle osseuse, lymphocytes du sang périphérique, cellules musculaires...), de les transfecter ou infecter *in vitro* selon les techniques de l'art et de les réadminister au patient. L'utilisation préférée est pour la préparation d'un médicament destiné au traitement des maladies par thérapie génique ou immunothérapie.

L'invention s'étend également à une méthode de traitement selon laquelle on administre une quantité thérapeutiquement efficace d'un vecteur adénoviral recombinant, d'une préparation virale ou d'une cellule hôte selon l'invention à un patient ayant besoin d'un tel traitement.

### EXEMPLES

La présente invention est illustrée, sans pour autant être limitée, par les exemples suivants.

Les constructions décrites ci-dessous sont réalisées selon les techniques générales de génie génétique et de clonage moléculaire, détaillées dans Maniatis et al., (1989, Laboratory Manual, Cold Spring Harbor, Laboratory Press, Cold Spring Harbor, NY) ou selon les recommandations du fabricant lorsqu'on utilise un kit commercial. Les étapes de recombinaison homologue sont de préférence réalisées dans la souche *E*. *coli* BJ 5183 (Hanahan, 1983, J. Mol. Biol. *166*, 557-580). S'agissant de la réparation des sites de restriction, la technique employée consiste en un remplissage des extrémités 5' protubérantes à l'aide du grand fragment de l'ADN polymérase I d'*E*. *coli* (Klenow). Par ailleurs, les fragments de génome adénoviral employés dans les différentes constructions décrites ci-après, sont indiqués précisément selon leur position dans la séquence nucléotidique du génome de l'Ad5 et de BAV3 telle que divulguée dans la banque de données Genebank sous la référence M73260 et AFO30154 respectivement.

En ce qui concerne la biologie cellulaire, les cellules sont transfectées ou transduites et cultivées selon les techniques standards bien connues de l'homme du métier. Dans les exemples qui suivent, on a recours aux lignées cellulaires 293 (Graham et al, 1977, *supra* ; disponible à l'ATCC sous la référence CRL1573) et MDBK (ATCC CRL-6071 ou CCL-22). Il est entendu que d'autres lignées cellulaires peuvent être utilisées.

### EXEMPLE 1 : Absence de multiplication des virus BAV3 dans les lignées humaines

La lignée humaine MDBK et les lignées humaines A549 (ATCC CCL-185), HeLa (ATCC CCL-2) et 293 (ATCC CRL-1573) sont infectées par un virus sauvage BAV3 à différentes MOI (1, 2 et 10). Les cellules sont récoltées 3 jours après l'infection et les titres viraux déterminés sur les cellules permissives MDBK. Le facteur de multiplication est inférieur à 1 dans le cas de l'infection des cellules humaines alors qu'il est compris entre 50 et 100 pour la lignée bovine. Ces résultats indiquent l'incapacité du vecteur BAV3 à se propager dans les lignées humaines.

L'expression des gènes viraux de BAV3 est vérifiée par PCR réverse après infection du virus BAV3 dans des lignées humaines établies (A549, HeLa, 293, MRC5, RPMI) ou primaires (lignée primaire de muscle PHM). La lignée contrôle est constituée par la lignée MDBK. Les cellules sont recueillies et on isole les ARN polyA+ selon les techniques conventionnelles. La transcription réverse est réalisée avec une amorce antisens spécifique de la région E1 et suivie d'une amplification par PCR avec la même amorce et une amorce sens spécifique de E1. Les produits PCR sont soumis à une analyse par Southem blot et détectés par une sonde spécifique de E1 permettant de détecter une bande de 626 pb correpondant à l'ADN génomique et de 519 pb dérivé de l'ARNm polyA+. Dans les cellules MDBK, on observe une expression du gène viral E1 qui est maximale 16 heures après l'infection. E1 est également exprimée dans la lignée 293. Par contre aucune expression d'ARNm E1 ne peut être détectée dans toutes les autres lignées humaines testées.

Ces résultats montrent que les cellules humaines sont infectées par le virus BAV3 mais que celui-ci ne peut pas s'y répliquer.

### EXEMPLE 2 : Construction de vecteurs adénoviraux chimères Ad5/BAV3

On construit tout d'abord un vecteur comprenant une cassette d'expression du gène bactérien LacZ codant pour la β-galactosidase. Pour ce faire, le fragment XhoI-SalI de pTG8595 (Lusky et al., 1998, J. Virol. *72*, 2022-2033) portant les séquences codantes LacZ est cloné dans le site XhoI du plasmide pCI (Promega). Ce dernier est un vecteur d'expression eucaryote comprenant le promoteur CMV, des séquences d'épissage, des sites multiples de clonage et la séquence polyA de SV40. La cassette d'expression du gène LacZ flanqué des éléments de régulation précités est isolée sous forme d'un fragment BglII-BamHI et introduit dans le site BglII du vecteur de transfert pTG8343, pour donner pTG6452. A titre indicatif, le vecteur de transfert comporte l'extrémité 5' de l'Ad5 délété de la majorité des séquences E1 (nt 1 à 458 et 3329 à 5788) insérée dans un plasmide ppolyII (Lathe et al., 1987, Gene 57, 193-201). Le génome adénoviral est reconstitué par recombinaison homologue entre le fragment PacI-NsiI obtenu de pTG6452 et le vecteur pTG3652 portant le génome Ad5 délété de la région E3 clivé par l'enzyme ClaI. Le vecteur ainsi obtenu, nommé pTG6481, contient les séquences Ad5 dépourvues des régions E1 (nt 459 à 3328) et E3 (nt 28249 à 30758) et la cassette pCMV-LacZ-pA SV40 clonée en lieu et place de la région E1. La région d'encapsidation est d'origine Ad5.

L'étape suivante est d'insérer la région d'encapsidation de BAV3 dans le vecteur précédent. Deux sites d'insertion ont été testés : le premier en amont de la région d'encapsidation native, au niveau du site AflIII en position 151 du génome Ad5 et l'autre en aval de celle-ci au niveau du site SalI (en position 451 de l'Ad5). La première construction pTG6466 est générée par digestion AflIII de pTG6452, traitement à la Klenow et introduction du fragment HaeII-PvuII de 844 pb recouvrant les séquences en positions 141 à 984 du génome BAV3, ce fragment ayant été soumis au préalable à l'action de la Klenow. Le vecteur pTG6467 est généré selon le même protocole mis à part que pTG6452 est linéarisé par SalI. Ainsi les vecteurs pTG6466 et pTG6467 contiennent deux régions d'encapsidation, l'une d'origine Ad5 et l'autre d'origine BAV3 (environ 0,8 kb).

On a également mis en oeuvre une région d'encapsidation BAV3 réduite issue de la précédente par digestion ThaI ou BstuI (positions 185 à 514 du génome BAV3). Comme précédemment, la région BAV3 d'environ 0,3 kb est insérée dans les vecteurs recombinants Ad5 soit au niveau du site AflIII (en 5' de la région psi autologue) en au niveau du site SalI (en 3' de la région psi autologue). Dans ce dernier cas, on génère le vecteur de transfert pTG6458 et le génome adénoviral est reconstitué par recombinaison homologue comme précédemment pour donner pTG6482.

Puis on génère une construction dans laquelle la région d'encapsidation d'Ad5 est échangée contre son homologue BAV3. Le vecteur pTG6452 est digéré par AflIIII (nt151) et SalI (nt 451) puis traité à la polymérase Klenow. Le fragment de 300 pb portant la région d'encapsidation Ad5 est remplacé par le fragment HaeII-PvuII (844 pb) isolé de BAV3 et rendu franc par l'action de la Klenow. On obtient pTG6468 qui porte la seule région d'encapsidation de BAV3 dans un contexte Ad5. Une construction identique met en oeuvre la région d'encapsidation BAV3 de 0,3 kb.

Les régions modifiées sont réintroduites dans le génome adénoviral par recombinaison homologue comme indiqué précédemment.

De manière identique, un vecteur adénoviral auxiliaire dérivé du génome de l'Ad 5 comportant la région d'encapsidation et les ITR 5' et 3' de BAV3 a été construit. Pour cela, le vecteur pTG13373 qui est dérivé du génome d'Ad5 délété des régions E1 et E3 est construit, comportant l'ITR 5' et la région d'encapsidation de 0,3 kb du génome de BAV3.

Le vecteur pTG 13373 est obtenu par le remplacement de l'ITR 5' et de la région d'encapsidation d'Ad5 présents dans pTG 8343, par l'ITR 5' et la région d'encapsidation de BAV 3 présents dans pTG 5431 ; à titre indicatif, pTG 5431 est constitué de l'extrémité 5' (nucléotides 1 à 8217) du génome de BAV 3. Pour ce faire, pTG 8343 est digéré par Bgl II, soumis à l'action de la Klenow, puis digéré par Pac I, et pTG 5431 est digéré par Acc I, traité à la Klenow, et digéré par Pac I. Les fragments ainsi obtenus sont liés pour donner pTG 13372. Finalement, pTG 13373 est obtenu par recombinaison homologue entre les fragments obtenus de pTG 13372 digéré par Bgl I et de pTG 6401 digéré par Pac I.

Le vecteur pTG 14310 dérivé du génome de l'Ad5 délété des régions E1 et E3 et comprenant les ITR 5' et 3' ainsi que la région d'encapsidation de BAV 3 est ensuite obtenu de la manière suivante.

On procède au remplacement de l'ITR 3' d'Ad5 dans le vecteur pTG 13384 (constitué par l'extrémité 3' (nucléotides 32800 à 35935) du génome de l'Ad 5 ; une cassette de clonage étant insérée entre les nucléotides 35826 et 35827) par l'ITR 3' de BAV 3. Pour ce faire, pTG 13384 est digéré par Xba I, soumis à l'action de la Klenow, puis digéré par Pac I. D'autre part, pTG 5451 est digéré par Apo I, traité à la Klenow, et digéré par Pac I. A titre indicatif, le vecteur pTG 5451 comprend les extrémités 5' (nucléotides 1 à 1651 délétés du fragment 829-1077) et 3' de BAV 3 (nucléotides 33232 à 34446) séparées par un site de restriction unique Hind III. Les fragments ainsi obtenus sont liés pour donner pTG 14261.

Par ailleurs, la recircularisation de pTG 14261 après digestion par XbaI et Bam HI et action de la Klenow permet l'élimination du site de clivage Hind III en amont de l'ITR bovin de pTG 14261. On obtient ainsi le vecteur pTG 14262.

Parallèlement, on procède à la recirculrisation de pTG 13373 digéré par Hind III pour obtenir pTG 14263 qui contient l'ITR 5' et la séquence d'encapsidation de 0,3 kb de BAV 3 ainsi que l'ITR 3' de l'Ad5.

On procède ensuite au remplacement de l'ITR 3' de l'Ad5 dans le vecteur pTG 14263 par l'ITR 3' de BAV 3 de pTG 14262. Pour ce faire, pTG 14263 et pTG 14262 sont digérés par Hind III et Pac I. Les fragments ainsi obtenus sont liés pour donner pTG 14266.

Enfin, le génome adénoviral est reconstitué par recombinaison homologue entre pTG 14266 linéarisé par Hind III et pTG 13373 digéré par Pac I. Le vecteur obtenu nommé pTG 14310 dérive du génome d'Ad 5 délété des régions E1 et E3 et contient les ITR 5' et 3' ainsi que la région d'encapsidation de BAV 3.

Un génome adénoviral similaire à celui de pTG 14310 mais comportant une région d'encapsidation de BAV3 de 0,8 kb a également été obtenu. On a pour cela procédé au remplacement de l'ITR 5' et de la région d'encapsidation d'Ad5 présents dans pTG 8343 par l'ITR 5' et la région d'encapsidation de 0,8 kb de BAV 3 présents dans pTG 5431. Pour ce faire, pTG 8343 est digéré par Bgl II, soumis à l'action de la Klenow, puis digéré par Pac I. D'autre part, pTG 5431 est digéré par Pvu II et Pac I. Les fragments ainsi obtenus sont liés pour donner pTG 14313.

On procède ensuite au remplacement de l'ITR 5' et de la région d'encapsidation de 0,3 kb de BAV 3 présents dans pTG 14266 par l'ITR 5' et la région d'encapsidation de 0,8 kb de BAV 3 présents dans pTG 14313. Pour ce faire, on procède à un recombinaison homologue entre les fragments Ssp I / Mfe I obtenu de pTG 14266 et Sca I / Nsi I obtenu de pTG 14313. Le vecteur ainsi obtenu est nommé pTG 14315.

Finalement le génome adénoviral est reconstitué par recombinaison homologue entre les fragments Hind III obtenu de pTG 14315 et Pac I obtenu de pTG 6401. Le vecteur ainsi obtenu, nommé pTG 14316, dérive du génome de l'Ad 5 et comporte les ITR 5' et 3' ainsi que la région d'encapsidation de 0,8 kb de BAV 3.

Un exemple supplémentaire de vecteur selon l'invention consiste en un vecteur dérivé du génome de l'Ad5 delété des régions E1 et E3 et contenant les ITR 5' et 3' ainsi que la région d'encapsidation de 0,3 kb de BAV 3, dans lequel la région d'encapsidation a été insérée juste en amont de l'ITR 3'.

Pour construire un tel vecteur, on procède tout d'abord à la délétion de la région d'encapsidation de BAV 3 dans pTG 13372 et dans pTG 14266. Pour cela, pTG 13372 est digéré par Afl III, soumis à l'action de la Klenow, puis digéré par Pac I et pTG 14262 est digéré par Pvu II et Pac I. Les fragments ainsi obtenus sont liés pour donner pTG 14311.

La délétion de la région d'encapsidation bovine de pTG 14266 est obtenue par recombinaison homologue entre le fragment Xmn I / Dra III obtenu de pTG 14266 et le fragment Pvu I / Sph I obtenu de pTG 14311. On obtient alors le vecteur pTG 14328.

On procède ensuite au remplacement de l'ITR 3' d'Ad5 dans pTG 13384 par l'ITR 3' et la séquence d'encapsidation de 0,3 kb de BAV 3 présents sur pTG 5431. Pour cela, pTG 13384 est digéré par Bam HI, soumis à l'action de la Klenow, puis digéré par Pac I. D'autre part, pTG 5431 est digéré par Afl III, traité à la Klenow, et digéré par Pac I. Les fragments ainsi obtenus sont liés pour donner pTG 14271.

L'introduction de la séquence d'encapsidation BAV 3 juste en amont de l'ITR 3' est réalisée par ligature des fragments Eco RI / Hind III obtenus de pTG14328 et de pTG 14271. Le vecteur ainsi obtenu est nommé pTG 14330.

Le génome adénoviral est ensuite reconstitué par recombinaison homologue entre les fragments Hind III de pTG 14330 et Pac I de pTG 6401. On obtient alors le vecteur pTG prod 11 qui dérive du génome de l'Ad5 delété des régions E1 et E3 et contient les ITR 5' et 3' ainsi que la région d'encapsidation de 0,3 kb de BAV 3 insérée juste en amont de l'ITR 3'.

### EXEMPLE 3 : Production de particules virales.

Les génomes viraux pTG6468 (auxiliaire) et pTG6467 ou pTG6466 (recombinant) sont transfectés dans les cellules bovines de la lignée MBDK. Puis, les cellules transfectées sont infectées par un génome BAV3 sauvage ou atténué. Puisque l'Ad5 peut se propager dans les cellules bovines en présence d'un virus BAV3 et que les trois éléments viraux contiennent une région d'encapsidation de BAV3, ils peuvent être empaquetés dans les capsides virales et générer des particules virales infectieuses. Le mélange est récupéré et on procède éventuellement à une étape d'amplification par cycles d'infection successifs de cellules MBDK afin de constituer un stock viral des trois types de virus. Lors de cette première étape sur cellules bovines, le génome BAV3 produit les facteurs agissant *en trans* pour l'encapsidation du vecteur auxiliaire lequel fournit les fonctions virales nécessaires à la propagation du vecteur recombinant. L'encapsidation de ce dernier peut être médiée par des facteurs adénoviraux d'origine BAV3 et Ad5 dans la mesure où il possède les régions d'encapsidation des deux origines et les capsides peuvent contenir des protéines structurales BAV3 ou Ad5.

Le mélange viral généré dans la lignée bovine est utilisé pour infecter des cellules humaines 293. Dans ces cellules, le virus BAV3 ne peut se propager même en présence d'Ad5. Cependant le vecteur auxiliaire d'origine Ad5 peut répliquer son génome viral et exprimer l'ensemble des gènes viraux précoces et tardifs qu'il porte. Par contre, il ne peut être encapsidé du fait qu'il est muni d'une seule région d'encapsidation BAV3. Au contraire, le vecteur recombinant peut l'être par l'intermédiaire de la région d'encapsidation d'origine Ad5 et des facteurs d'encapsidation fournis par le vecteur auxiliaire. Les particules virales générées sont récupérées et peuvent être utilisées à des fins thérapeutiques.

## Revendications

1. Vecteur adénoviral dérivant d'un génome adénoviral **caractérisé en ce qu'**il comprend une région essentielle à l'encapsidation d'origine adénovirale et hétérologue par rapport audit génome adénoviral.

2. Vecteur adénoviral selon la revendication 1, **caractérisé en ce qu'**il dérive d'un adénovirus d'origine humaine et que ladite région essentielle à l'encapsidation hétérologue dérive d'un adénovirus d'origine animale.

3. Vecteur adénoviral selon la revendication 2, **caractérisé en ce qu'**il dérive d'un adénovirus humain du sous groupe C et notamment d'un adénovirus 2 (Ad2) ou 5 (Ad5).

4. Vecteur adénoviral selon l'une des revendications 1 à 3, **caractérisé en ce que** ladite région essentielle à l'encapsidation hétérologue dérive d'un adénovirus animal sélectionné parmi les adénovirus canins, aviaires, bovins, murins, ovins, porcins et simiens.

5. Vecteur adénoviral selon la revendication 3 ou 4, **caractérisé en ce qu'**il dérive d'un Ad5 et que ladite région essentielle à l'encapsidation hétérologue dérive d'un adénovirus bovin.

6. Vecteur adénoviral selon la revendication 5, **caractérisé en ce que** ledit adénovirus bovin est BAV3.

7. Vecteur adénoviral selon l'un des revendication 1 à 6, **caractérisé en ce que** ladite région essentielle à l'encapsidation comprend la région d'encapsidation.

8. Vecteur adénoviral selon la revendication 7, **caractérisé en ce que** ladite région essentielle à l'encapsidation comprend en outre les ITR 5' et 3'.

9. Vecteur adénoviral selon l'une des revendications 1 à 8, **caractérisé en ce qu'**il est défectif au moins pour la fonction E1.

10. Vecteur adénoviral selon la revendication 9, **caractérisé en ce qu'**il est en outre défectif pour l'une au moins des fonctions sélectionnées parmi les fonctions E2, E3, E4 et L1-L5.

11. Vecteur adénoviral selon l'une des revendications 1 à 10, **caractérisé en ce qu'**il s'agit d'un vecteur auxiliaire permettant de complémenter tout ou partie des fonctions défectives d'un vecteur adénoviral recombinant.

12. Vecteur adénoviral auxiliaire selon la revendication 11, **caractérisé en ce qu'**il comprend les séquences ITR 5' et 3' et les séquences codant pour les fonctions E2, E4 et/ou L1-L5 dérivant d'un adénovirus humain,et une région d' encapsidation hétérologue dérivant d'un adénovirus bovin.

13. Vecteur adénoviral auxiliaire selon la revendication 11, **caractérisé en ce qu'**il comprend les séquences codant pour les fonctions E2, E4 et/ou L1-L5 dérivant d'un adénovirus humain, et les séquences ITR 5' et 3' et la région d' encapsidation hétérologue dérivant d'un adénovirus bovin.

14. Vecteur adénoviral selon l'une des revendications 1 à 10, **caractérisé en ce qu'**il est recombinant et comprend au moins un gène d'intérêt placé sous le contrôle des éléments nécessaires à son expression dans une cellule ou un organisme hôte.

15. Vecteur adénoviral recombinant selon la revendication 14, **caractérisé en ce que** ledit gène d'intérêt est sélectionné parmi les gènes codant pour un ARN antisens, une cytokine, un récepteur cellulaire ou nucléaire, un ligand, un facteur de coagulation, la protéine CFTR, l'insuline, la dystrophine, un facteur de croissance, une enzyme, un inhibiteur d'enzyme, un polypeptide à effet anti-tumoral, un polypeptide capable d'inhiber une infection bactérienne, parasitaire ou virale et, notamment le VIH, un anticorps, une toxine, une immunotoxine, un inhibiteur d'angiogénèse et un marqueur.

16. Vecteur adénoviral recombinant selon la revendication 14 ou 15, **caractérisé en ce qu'**il comprend en outre une seconde région d'encapsidation autologue par rapport au génome adénoviral dont il dérive.

17. Vecteur adénoviral recombinant selon la revendication 16, **caractérisé en ce qu'**il est défectif pour l'ensemble des fonctions adénovirales et comprend outre ledit gène d'intérêt, au moins les ITRs 5' et 3' et ladite région d'encapsidation autologue dérivant d'un adénovirus humain, et ladite région d'encapsidation hétérologue dérivant d'un adénovirus bovin.

18. Vecteur adénoviral recombinant selon la revendication 16 ou 17, **caractérisé en ce que** la région d'encapsidation hétérologue est insérée en 3' de la région d'encapsidation autologue.

19. Utilisation d'un vecteur adénoviral auxiliaire selon l'une des revendications 11 à 13 pour complémenter tout ou partie des fonctions défectives d'un vecteur adénoviral recombinant défectif pour la réplication, lesdits vecteurs auxiliaire et recombinant dérivant d'un même premier adénovirus et, en particulier d'un Ad5 et lesdites régions essentielles à l'encapsidation hétérologues portées par le vecteur auxiliaire dérivant d'un second adénovirus différent du premier et, en particulier d'un BAV3.

20. Utilisation selon la revendication 19 **caractérisée en ce que** le vecteur adénoviral recombinant défectif pour la réplication est un vecteur selon l'une des revendications 1 à 10 et 14 à 18, lesdits vecteurs auxiliaire et recombinant dérivant d'un même premier adénovirus et lesdites régions essentielles à l'encapsidation hétérologues portées par le vecteur auxiliaire et ledit vecteur recombinant dérivant d'un second adénovirus différent du premier.

21. Composition comprenant :
(a) un vecteur adénoviral auxiliaire selon l'une des revendications 11 à 13,
(b) un vecteur adénoviral recombinant défectif pour la réplication, et
(c) de manière optionnelle un génome adénoviral dérivant d'un adénovirus animal de la même origine que les régions essentielles à l'encapsidation hétérologues portées par le vecteur a).

22. Composition selon la revendication 21 **caractérisée en ce que** ledit vecteur recombinant adénoviral défectif pour la réplication (b) est un vecteur adénoviral recombinant selon l'une des revendications 1 à 10 et 14 à 18 et **en ce que** l'on a en (c) de manière optionnelle, un génome adénoviral dérivant d'un adénovirus animal de la même origine que les régions essentielles à l'encapsidation hétérologues portées par les vecteurs a) et b).

23. Procédé pour préparer une préparation virale comprenant un vecteur adénoviral recombinant défectif pour la réplication, selon lequel :
(a) on introduit dans une première lignée cellulaire
(i) un vecteur adénoviral auxiliaire selon l'une des revendications 11 à 13,
(ii) un génome adénoviral dérivant d'un adénovirus animal, et
(iii) ledit vecteur adénoviral recombinant,
ledit génome adénoviral (ii) étant de la même origine que les régions essentielles à l'encapsidation hétérologues portées par le vecteur (i) et ledit génome adénoviral (ii) et les vecteurs (i) et (iii) étant capables de se répliquer dans ladite première lignée cellulaire,
(b) on cultive ladite première lignée cellulaire dans des conditions appropriées pour permettre la production de particules virales comportant les vecteurs (i) et (iii) et le génome adénoviral (ii),
(c) on récupère lesdites particules virales obtenues à l'étape b) dans la culture cellulaire,
(d) on infecte une seconde lignée cellulaire avec lesdites particules virales récupérées à l'étape c), ledit vecteur auxiliaire (i) et ledit génome adénoviral (ii) ayant une capacité d'encapsidation et/ou de réplication nulle ou réduite dans ladite seconde lignée cellulaire,
(e) on cultive ladite seconde lignée cellulaire dans des conditions appropriées pour permettre l'encapsidation dudit vecteur adénoviral recombinant (iii) et produire ladite préparation virale, et
(f) on récupère ladite préparation virale obtenue à l'étape e) dans la culture cellulaire.

24. Procédé selon la revendication 23 **caractérisé en ce que** ledit vecteur adénoviral recombinant défectif pour la réplication est un vecteur selon l'une des revendications 1 à 10 et 14 à 18 et **en ce que** ledit génome adénoviral (ii) est de la même origine que les régions essentielles à l'encapsidation hétérologues portées par les vecteurs (i) et (iii) et ledit génome adénoviral (ii), et les vecteurs (i) et (iii) sont capables de se répliquer dans ladite première lignée cellulaire.

25. Procédé selon la revendication 24, selon lequel on introduit dans ladite première lignée cellulaire :
(i) un vecteur adénoviral auxiliaire selon l'une des revendications 11-13 défectif au moins pour la fonction E1,
(ii) un génome adénoviral dérivant d'un adénovirus bovin, notamment d'un BAV3, éventuellement présentant une capacité d'encapsidation atténuée par rapport à l'adénovirus dont il dérive, et
(iii) un vecteur adénoviral recombinant défectif pour la fonction E1 et au moins l'une des fonctions E2, E4 et/ou L1-L5, notamment un vecteur selon l'une des revendications 14 à 18,
ladite première lignée cellulaire étant d'origine bovine.

26. Procédé selon l'une des revendications 23 à 25, selon lequel ledit génome adénoviral (ii) est défectif pour la fonction E1 et ladite première lignée cellulaire est une cellule de complémentation de la fonction E1 dudit génome adénoviral (ii).

27. Procédé selon la revendication 26, selon lequel ladite première lignée cellulaire dérive d'une lignée MDBK ou de cellules primaires bovines de rétine ou de rein foetal et comprend les séquences codant pour la région E1 d'un adénovirus bovin et en particulier d'un BAV3 placées sous le contrôle des éléments nécessaires à leur expression.

28. Procédé selon l'une des revendications 23 à 27, selon lequel ladite seconde lignée cellulaire est une cellule de complémentation de la fonction E1 d'un adénovirus humain, notamment d'un Ad5.

29. Procédé selon la revendication 28, selon lequel ladite seconde lignée cellulaire est la lignée 293.

30. Préparation virale susceptible d'être obtenue par le procédé selon l'une des revendications 23 à 29.

31. Préparation virale selon la revendication 30, comprenant au moins 30 % de particules virales infectieuses contenant un vecteur adénoviral recombinant selon l'une des revendications 1 à 10 et 14 à 18.

32. Cellule hôte comprenant un vecteur adénoviral selon l'une des revendications 1 à 18 ou infectée par une préparation virale selon la revendication 30 ou 31.

33. Cellule comprenant :
(i) un vecteur adénoviral auxiliaire selon l'une des revendications 11 à 13, et
(ii) un génome adénoviral dérivant d'un adénovirus animal.

34. Cellule selon la revendication 33 renfermant en outre (iii) un vecteur adénoviral recombinant défectif, et notamment un vecteur selon l'une des revendications 1 à 10 et 14 à 18,

35. Composition pharmaceutique comprenant à titre d'agent thérapeutique ou prophylactique un vecteur adénoviral selon l'une des revendications 1 à 10 et 14 à 18, une préparation virale selon la revendication 30 ou 31 ou une cellule hôte selon la revendication 32.

36. Utilisation d'un vecteur adénoviral selon l'une des revendications 1 à 10 et 14 à 18, d'une préparation virale selon la revendication 30 ou 31 ou d'une cellule hôte selon la revendication 32, pour la préparation d'un médicament destiné au traitement préventif ou curatif de maladies genétiques telles que l'hémophilie, le diabète, la mucoviscidose, la myopathie de Duchenne ou de Becker, de maladies auto-immunes ou de maladies acquises telles que les cancers, les tumeurs, les maladies cardiovasculaires, la maladies d'origine infectieuses comme l'hepatite B ou C et le SIDA par transfert et expression d'un gène d'intérêt dans une cellule ou un organisme hôte.

37. Utilisation d'un vecteur adénoviral selon l'une des revendications revendications 1 à 10 et 14 à 18, d'une préparation virale selon la revendication 30 ou 31 ou d'une cellule hôte selon la revendication 33, pour la préparation d'un médicament destiné au traitement des maladies par thérapie génique ou immunothérapie.

## Patentansprüche

1. Adenoviraler Vektor, welcher sich von einem adenoviralen Genom ableitet, **dadurch gekennzeichnet, dass** er eine für die Verkapselung essentielle Region von adenoviraler Herkunft, welche in Bezug auf das adenovirale Genom heterolog ist, umfasst.

2. Adenoviraler Vektor nach Anspruch 1, **dadurch gekennzeichnet, dass** er sich von einem Adenovirus von humaner Herkunft ableitet und dass die für die Verkapselung essentielle heterologe Region sich von einem Adenovirus von tierischer Herkunft ableitet.

3. Adenoviraler Vektor nach Anspruch 2, **dadurch gekennzeichnet, dass** er sich von einem humanen Adenovirus der Untergruppe C und insbesondere von einem Adenovirus 2 (Ad2) oder 5 (Ad5) ableitet.

4. Adenoviraler Vektor nach einem der Ansprüche 1 bis 3, **dadurch gekennzeichnet, dass** die für die Verkapselung essentielle heterologe Region sich von einem tierischen Adenovirus, ausgewählt unter den Hunde-, Vögel-, Rinder-, Mäuse-, Schaf-, Schweine- und Affen-Adenoviren, ableitet.

5. Adenoviraler Vektor nach Anspruch 3 oder 4, **dadurch gekennzeichnet, dass** er sich von einem Ad5 ableitet und dass die für die Verkapselung essentielle heterologe Region sich von einem Rinder-Adenovirus ableitet.

6. Adenoviraler Vektor nach Anspruch 5, **dadurch gekennzeichnet, dass** das Rinder-Adenovirus BAV3 ist.

7. Adenoviraler Vektor nach einem der Ansprüche 1 bis 6, **dadurch gekennzeichnet, dass** die für die Verkapselung essentielle Region die Verkapselungsregion umfasst.

8. Adenoviraler Vektor nach Anspruch 7, **dadurch gekennzeichnet, dass** die für die Verkapselung essentielle Region außerdem die 5'- und 3'-ITR umfasst.

9. Adenoviraler Vektor nach einem der Ansprüche 1 bis 8, **dadurch gekennzeichnet, dass** er einen Mangel wenigstens hinsichtlich der E1-Funktion aufweist.

10. Adenoviraler Vektor nach Anspruch 9, **dadurch gekennzeichnet, dass** er außerdem einen Mangel wenigstens hinsichtlich einer der unter den Funktionen E2, E3, E4 und L1-L5 ausgewählten Funktionen aufweist.

11. Adenoviraler Vektor nach einem der Ansprüche 1 bis 10, **dadurch gekennzeichnet, dass** es sich um einen Hilfsvektor handelt, welcher ermöglicht, die Gesamtheit oder einen Teil der mangelhaften bzw. fehlenden Funktionen eines rekombinanten adenoviralen Vektors zu komplementieren.

12. Adenoviraler Hilfsvektor nach Anspruch 11, **dadurch gekennzeichnet, dass** er die 5'- und 3'-ITR-Sequenzen und die Sequenzen, die die Funktionen E2, E4 und/oder L1-L5 kodieren, welche sich von einem humanen Adenovirus ableiten, und eine heterologe Verkapselungsregion, welche sich von einem Rinder-Adenovirus ableitet, umfasst.

13. Adenoviraler Hilfsvektor nach Anspruch 11, **dadurch gekennzeichnet, dass** er die die Funktionen E2, E4 und/oder L1-L5 kodierenden Sequenzen, welche sich von einem humanen Adenovirus ableiten, und die 5'- und 3'-ITR-Sequenzen und die Verkapselungsregion, welche sich von einem Rinder-Adenovirus ableiten, umfasst.

14. Adenoviraler Vektor nach einem der Ansprüche 1 bis 10, **dadurch gekennzeichnet, dass** er rekombinant ist und wenigstens ein Gen von Interesse, welches unter die Kontrolle der für dessen Expression in einer Zelle oder einem Wirtsorganismus erforderlichen Elemente gestellt ist, umfasst.

15. Rekombinanter adenoviraler Vektor nach Anspruch 14, **dadurch gekennzeichnet, dass** das Gen von Interesse ausgewählt wird unter den Genen, welche eine Antisinn-RNA, ein Zytokin, einen zellulären oder nukleären Rezeptor, einen Liganden, einen Gerinnungsfaktor, das CFTR-Protein, Insulin, Dystrophin, einen Wachstumsfaktor, ein Enzym, einen Enzyminhibitor, ein Polypeptid mit Antitumor-Wirkung, ein Polypeptid, welches in der Lage ist, eine bakterielle, durch Parasiten hervorgerufene oder virale Infektion und insbesondere HIV zu hemmen, einen Antikörper, ein Toxin, ein Immuntoxin, einen Inhibitor der Angiogenese und einen Marker kodieren.

16. Rekombinanter adenoviraler Vektor nach Anspruch 14 oder 15, **dadurch gekennzeichnet, dass** er außerdem eine zweite Verkapselungsregion umfasst, welche in Bezug auf das adenovirale Genom, von welchem er sich ableitet, autolog ist.

17. Rekombinanter adenoviraler Vektor nach Anspruch 16, **dadurch gekennzeichnet, dass** er einen Mangel hinsichtlich der Gesamtheit der adenoviralen Funktionen aufweist und außer dem Gen von Interesse wenigstens die 5'- und 3'-ITRs und die autologe Verkapselungsregion, welche sich von einem humanen Adenovirus ableitet, und die heterologe Verkapselungsregion, welche sich von einem Rinder-Adenovirus ableitet, umfasst.

18. Rekombinanter adenoviraler Vektor nach Anspruch 16 oder 17, **dadurch gekennzeichnet, dass** die heterologe Verkapselungsregion 3' von der autologen Verkapselungsregion inseriert ist.

19. Verwendung eines adenoviralen Hilfsvektors nach einem der Ansprüche 11 bis 13, um die Gesamtheit oder einen Teil der mangelhaften oder fehlenden Funktionen eines rekombinanten adenoviralen Vektors, welcher einen Mangel hinsichtlich der Replikation aufweist, zu komplementieren, wobei der Hilfsvektor und der rekombinante Vektor sich von dem gleichen ersten Adenovirus und insbesondere von einem Ad5 ableiten und die für die Verkapselung essentiellen heterologen Regionen, welche der Hilfsvektor enthält, sich von einem zweiten Adenovirus, welches sich von dem ersten unterscheidet, und insbesondere von einem BAV3 ableiten.

20. Verwendung nach Anspruch 19, **dadurch gekennzeichnet, dass** der rekombinante adenovirale Vektor, welcher einen Mangel hinsichtlich der Replikation aufweist, ein Vektor nach einem der Ansprüche 1 bis 10 und 14 bis 18 ist, wobei der Hilfsvektor und der rekombinante Vektor sich von dem gleichen ersten Adenovirus ableiten und die für die Verkapselung essentiellen heterologen Regionen, welche der Hilfsvektor und der rekombinante Vektor enthalten, sich von einem zweiten Adenovirus, welches sich von dem ersten unterscheidet, ableiten.

21. Zusammensetzung, umfassend:
(a) einen adenoviralen Hilfsvektor nach einem der Ansprüche 11 bis 13,
(b) einen rekombinanten adenoviralen Vektor, welcher einen Mangel hinsichtlich der Replikation aufweist, und
(c) gegebenenfalls ein adenovirales Genom, welches sich von einem tierischen Adenovirus von der gleichen Herkunft wie die für die Verkapselung essentiellen heterologen Regionen, welche der Vektor a) enthält, ableitet.

22. Zusammensetzung nach Anspruch 21, **dadurch gekennzeichnet, dass** der rekombinante adenovirale Vektor, welcher einen Mangel hinsichtlich der Replikation aufweist, (b) ein rekombinanter adenoviraler Vektor nach einem der Ansprüche 1 bis 10 und 14 bis 18 ist und dass man in (c) gegebenenfalls ein adenovirales Genom, welches sich von einem tierischen Adenovirus von der gleichen Herkunft wie die für die Verkapselung essentiellen heterologen Regionen, welche die Vektoren a) und b) enthalten, ableitet, hat.

23. Verfahren zur Herstellung eines Viruspräparats, umfassend einen rekombinanten adenoviralen Vektor, welcher einen Mangel hinsichtlich der Replikation aufweist, gemäß welchem:
(a) man in eine erste Zelllinie einführt:
(i) einen adenoviralen Hilfsvektor nach einem der Ansprüche 11 bis 13,
(ii) ein adenovirales Genom, welches sich von einem tierischen Adenovirus ableitet, und
(iii) den rekombinanten adenoviralen Vektor,
wobei das adenovirale Genom (ii) von der gleichen Herkunft ist wie die für die Verkapselung essentiellen heterologen Regionen, welche der Vektor (i) enthält, und das adenovirale Genom (ii) und die Vektoren (i) und (iii) in der Lage sind, sich in der ersten Zelllinie zu replizieren,
(b) man die erste Zelllinie unter geeigneten Bedingungen kultiviert, um die Produktion von Viruspartikeln, welche die Vektoren (i) und (iii) und das adenovirale Genom (ii) umfassen, zu erlauben,
(c) man die in Schritt b) in der Zellkultur erhaltenen Viruspartikel gewinnt,
(d) man eine zweite Zelllinie mit den in Schritt c) gewonnenen Viruspartikeln infiziert, wobei der Hilfsvektor (i) und das adenovirale Genom (ii) keinerlei oder eine verringerte Fähigkeit zur Verkapselung und/oder Replikation in der zweiten Zelllinie aufweisen,
(e) man die zweite Zelllinie unter geeigneten Bedingungen kultiviert, um die Verkapselung des rekombinanten adenoviralen Vektors (iii) zu erlauben und das Viruspräparat zu produzieren, und
(f) man das in Schritt e) in der Zellkultur erhaltene Viruspräparat gewinnt.

24. Verfahren nach Anspruch 23, **dadurch gekennzeichnet, dass** der rekombinante adenovirale Vektor, welcher einen Mangel hinsichtlich der Replikation aufweist, ein Vektor nach einem der Ansprüche 1 bis 10 und 14 bis 18 ist und dass das adenovirale Genom (ii) von der gleichen Herkunft wie die für die Verkapselung essentiellen heterologen Regionen, welche die Vektoren (i) und (iii) und das adenovirale Genom (ii) enthalten, ist und die Vektoren (i) und (iii) in der Lage sind, sich in der ersten Zelllinie zu replizieren.

25. Verfahren nach Anspruch 24, gemäß welchem man in die erste Zelllinie einführt:
(i) einen adenoviralen Hilfsvektor nach einem der Ansprüche 11-13, welcher einen Mangel hinsichtlich wenigstens der Funktion E1 aufweist,
(ii) ein adenovirales Genom, welches sich von einem Rinder-Adenovirus, insbesondere einem BAV3, welches gegebenenfalls ein bezogen auf das Adenovirus, von welchem es sich ableitet, abgeschwächtes Verkapselungsvermögen aufweist, ableitet, und
(iii) einen rekombinanten adenoviralen Vektor, welcher einen Mangel hinsichtlich der Funktion E1 und wenigstens einer der Funktionen E2, E4 und/oder L1-L5 aufweist, insbesondere einen Vektor nach einem der Ansprüche 14 bis 18,
wobei die erste Zelllinie von Rinder-Herkunft ist.

26. Verfahren nach einem der Ansprüche 23 bis 25, gemäß welchem das adenovirale Genom (ii) einen Mangel hinsichtlich der Funktion E1 aufweist und die erste Zelllinie eine Zelle ist, welche zur Komplementierung der Funktion E1 des adenoviralen Genoms (ii) in der Lage ist.

27. Verfahren nach Anspruch 26, gemäß welchem die erste Zelllinie sich von einer Linie MDBK oder von primären Retina- oder fötalen Nierenzellen vom Rind ableitet und die Sequenzen, welche die Region E1 eines Rinder-Adenovirus und insbesondere eines BAV3 kodieren, welche unter die Kontrolle der für deren Expression erforderlichen Elemente gestellt sind, umfasst.

28. Verfahren nach einem der Ansprüche 23 bis 27, gemäß welchem die zweite Zelllinie eine Zelle ist, welche zur Komplementierung der Funktion E1 eines humanen Adenovirus, insbesondere eines Ad5, in der Lage ist.

29. Verfahren nach Anspruch 28, gemäß welchem die zweite Zelllinie die Linie 293 ist.

30. Viruspräparat, welches durch das Verfahren nach einem der Ansprüche 23 bis 29 erhalten werden kann.

31. Viruspräparat nach Anspruch 30, welches wenigstens 30% infektiöse Viruspartikel, welche einen rekombinanten adenoviralen Vektor nach einem der Ansprüche 1 bis 10 und 14 bis 18 enthalten, umfasst.

32. Wirtszelle, welche einen adenoviralen Vektor nach einem der Ansprüche 1 bis 18 umfasst oder durch ein Viruspräparat nach Anspruch 30 oder 31 infiziert ist.

33. Zelle, umfassend:
(i) einen adenoviralen Hilfsvektor nach einem der Ansprüche 11 bis 13 und
(ii) ein adenovirales Genom, welches sich von einem tierischen Adenovirus ableitet.

34. Zelle nach Anspruch 33, welche außerdem (iii) einen einen Mangel aufweisenden rekombinanten adenoviralen Vektor und insbesondere einen Vektor nach einem der Ansprüche 1 bis 10 und 14 bis 18 umfasst.

35. Pharmazeutische Zusammensetzung, welche als therapeutisches oder prophylaktisches Mittel einen adenoviralen Vektor nach einem der Ansprüche 1 bis 10 und 14 bis 18, ein Viruspräparat nach Anspruch 30 oder 31 oder eine Wirtszelle nach Anspruch 32 umfasst.

36. Verwendung eines adenoviralen Vektors nach einem der Ansprüche 1 bis 10 und 14 bis 18, eines Viruspräparats nach Anspruch 30 oder 31 oder einer Wirtszelle nach Anspruch 32 für die Herstellung eines Arzneimittels, welches für die präventive oder heilende Behandlung von genetisch bedingten Erkrankungen, wie Hämophilie, Diabetes, Mukoviszidose, Duchenne- oder Becker-Muskeldystrophie, von Autoimmunerkrankungen oder erworbenen Erkrankungen, wie Krebserkrankungen, Tumoren, Herz-Kreislauf-Erkrankungen, Krankheiten mit Infektions-Ursprung, wie Hepatitis B oder C und AIDS, bestimmt ist, durch Transfer und Expression eines Gens von Interesse in eine(r) Zelle oder einen/einem Wirtsorganismus.

37. Verwendung eines adenoviralen Vektors nach einem der Ansprüche 1 bis 10 und 14 bis 18, eines Viruspräparats nach Anspruch 30 oder 31 oder einer Wirtszelle nach Anspruch 33 für die Herstellung eines Arzneimittels, welches für die Behandlung der Erkrankungen durch Gentherapie oder Immuntherapie bestimmt ist.

## Claims

1. Adenoviral vector which is derived from an adenoviral genome, **characterized in that** it comprises a region essential for encapsidation which is of adenoviral origin and is heterologous with respect to said adenoviral genome.

2. Adenoviral vector according to Claim 1, **characterized in that** it is derived from an adenovirus of human origin and **in that** said heterologous region essential for encapsidation is derived from an adenovirus of animal origin.

3. Adenoviral vector according to Claim 2, **characterized in that** it is derived from a subgroup C human adenovirus and in particular from an adenovirus 2 (Ad2) or 5 (Ad5).

4. Adenoviral vector according to one of Claims 1 to 3, **characterized in that** said heterologous region essential for encapsidation is derived from an animal adenovirus selected from canine, avian, bovine, murine, ovine, porcine and simian adenoviruses.

5. Adenoviral vector according to Claim 3 or 4, **characterized in that** it is derived from an Ad5 and **in that** said heterologous region essential for encapsidation is derived from a bovine adenovirus.

6. Adenoviral vector according to Claim 5, **characterized in that** said bovine adenovirus is BAV3.

7. Adenoviral vector according to one of Claims 1 to 6, **characterized in that** said region essential for encapsidation comprises the encapsidation region.

8. Adenoviral vector according to Claim 7, **characterized in that** said region essential for encapsidation comprises, in addition, the 5' and 3' ITRs.

9. Adenoviral vector according to one of Claims 1 to 8, **characterized in that** it is defective at least for the E1 function.

10. Adenoviral vector according to Claim 9, **characterized in that** it is, in addition, defective for at least one of the functions selected from the E2, E3, E4 and L1-L5 functions.

11. Adenoviral vector according to one of Claims 1 to 10, **characterized in that** it is a helper vector which makes it possible to complement all or part of the defective functions of a recombinant adenoviral vector.

12. Helper adenoviral vector according to Claim 11, **characterized in that** it comprises the 5' and 3' ITR sequences and the sequences encoding the E2, E4 and/or L1-L5 functions derived from a human adenovirus, and a heterologous encapsidation region derived from a bovine adenovirus.

13. Helper adenoviral vector according to Claim 11, **characterized in that** it comprises the sequences encoding the E2, E4 and/or L1-L5 functions derived from a human adenovirus, and the 5' and 3' ITR sequences and the heterologous encapsidation region derived from a bovine adenovirus.

14. Adenoviral vector according to one of Claims 1 to 10, **characterized in that** it is recombinant and comprises at least one gene of interest placed under the control of elements necessary for its expression in a host cell or organism.

15. Recombinant adenoviral vector according to Claim 14, **characterized in that** said gene of interest is selected from the genes encoding an antisense RNA, a cytokine, a cell or nuclear receptor, a ligand, a coagulation factor, the CFTR protein, insulin, dystrophin, a growth factor, an enzyme, an enzyme inhibitor, a polypeptide with antitumor effect, a polypeptide capable of inhibiting a bacterial, parasitic or viral infection and, in particular HIV, an antibody, a toxin, an immunotoxin, an angiogenesis inhibitor and a marker.

16. Recombinant adenoviral vector according to Claim 14 or 15, **characterized in that** it comprises, in addition, a second encapsidation region which is autologous with respect to the adenoviral genome from which it is derived.

17. Recombinant adenoviral vector according to Claim 16, **characterized in that** it is defective for all the adenoviral functions and comprises, in addition to said gene of interest, at least the 5' and 3' ITRs and said autologous encapsidation region being derived from a human adenovirus, and said heterologous encapsidation region being derived from a bovine adenovirus.

18. Recombinant adenoviral vector according to Claim 16 or 17, **characterized in that** the heterologous encapsidation region is inserted in 3' of the autologous encapsidation region.

19. Use of a helper adenoviral vector according to one of Claims 11 to 13 for complementing all or part of the defective functions of a replication-defective recombinant adenoviral vector, said helper and recombinant vectors being derived from the same first adenovirus, and in particular from an Ad5, and said heterologous regions essential for encapsidation carried by the helper vector being derived from a second adenovirus different from the first, and in particular from a BAV3.

20. Use according to Claim 19, **characterized in that** the replication-defective recombinant adenoviral vector is a vector according to one of Claims 1 to 10 and 14 to 18, said helper and recombinant vectors being derived from the same first adenovirus and said heterologous regions essential for encapsidation carried by the helper vector and said recombinant vector being derived from a second adenovirus different from the first.

21. Composition comprising:
(a) a helper adenoviral vector according to one of Claims 11 to 13,
(b) a replication-defective recombinant adenoviral vector, and
(c) optionally, an adenoviral genome which is derived from an animal adenovirus of the same origin as the heterologous regions essential for encapsidation carried by the vector a).

22. Composition according to Claim 21, **characterized in that** said replication-defective recombinant adenoviral vector (b) is a recombinant adenoviral vector according to one of Claims 1 to 10 and 14 to 18 and **in that** an adenoviral genome which is derived from an animal adenovirus of the same origin as the heterologous regions essential for encapsidation carried by the vectors a) and b) is optionally present in (c).

23. Method for preparing a viral preparation comprising a replication-defective recombinant adenoviral vector, according to which:
(a) there are introduced into a first cell line
(i) a helper adenoviral vector according to one of Claims 11 to 13,
(ii) an adenoviral genome which is derived from an animal adenovirus, and
(iii) said recombinant adenoviral vector, said adenoviral genome (ii) being of the same origin as the heterologous regions essential for encapsidation which are carried by the vector (i) and said adenoviral genome (ii) and the vectors (i) and (iii) being capable of replicating in said first cell line,
(b) said first cell line is cultured under appropriate conditions to allow the production of viral particles comprising the vectors (i) and (iii) and the adenoviral genome (ii),
(c) said viral particles obtained in step b) are recovered from the cell culture,
(d) a second cell line is infected with said viral particles recovered in step c), said helper vector (i) and said adenoviral genome (ii) having a zero or reduced encapsidation and/or replication capacity in said second cell line,
(e) said second cell line is cultured under appropriate conditions to allow the encapsidation of said recombinant adenoviral vector (iii) and produce said viral preparation, and
(f) said viral preparation obtained in step e) is recovered from the cell culture.

24. Method according to Claim 23, **characterized in that** said replication-defective recombinant adenoviral vector is a vector according to one of Claims 1 to 10 and 14 to 18 and **in that** said adenoviral genome (ii) is of the same origin as the heterologous regions essential for encapsidation carried by the vectors (i) and (iii) and said adenoviral genome (ii), and the vectors (i) and (iii) are capable of replicating in said first cell line.

25. Method according to Claim 24, according to which there are introduced into said first cell line:
(i) a helper adenoviral vector according to one of Claims 11-13 which is defective at least for the E1 function,
(ii) an adenoviral genome derived from a bovine adenovirus, in particular from a BAV3, optionally having an attenuated encapsidation capacity compared with the adenovirus from which it is derived, and
(iii) a recombinant adenoviral vector which is defective for the E1 function and at least one of the E2, E4 and/or L1-L5 functions, in particular a vector according to one of Claims 14 to 18,
said first cell line being of bovine origin.

26. Method according to one of Claims 23 to 25, according to which said adenoviral genome (ii) is defective for the E1 function and said first cell line is a cell for complementing the E1 function of said adenoviral genome (ii).

27. Method according to Claim 26, according to which said first cell line is derived from an MDBK line or from primary bovine cells of the retina or of fetal kidney and comprises the sequences encoding the E1 region of a bovine adenovirus, and in particular of a BAV3, which are placed under the control of the elements necessary for their expression.

28. Method according to one of Claims 23 to 27, according to which said second cell line is a cell for complementing the E1 function of a human adenovirus, in particular of an Ad5.

29. Method according to Claim 28, according to which said second cell line is the 293 line.

30. Viral preparation which can be obtained by the method according to one of Claims 23 to 29.

31. Viral preparation according to Claim 30, comprising at least 30% of infectious viral particles containing a recombinant adenoviral vector according to one of Claims 1 to 10 and 14 to 18.

32. Host cell comprising an adenoviral vector according to one of Claims 1 to 18 or infected with a viral preparation according to Claim 30 or 31.

33. Cell comprising:
(i) a helper adenoviral vector according to one of Claims 11 to 13, and
(ii) an adenoviral genome which is derived from an animal adenovirus.

34. Cell according to Claim 33 containing, in addition, (iii) a defective recombinant adenoviral vector, and in particular a vector according to one of Claims 1 to 10 and 14 to 18.

35. Pharmaceutical composition comprising, as therapeutic or prophylactic agent, an adenoviral vector according to one of Claims 1 to 10 and 14 to 18, a viral preparation according to Claim 30 or 31 or a host cell according to Claim 32.

36. Use of an adenoviral vector according to one of Claims 1 to 10 and 14 to 18, of a viral preparation according to Claim 30 or 31 or of a host cell according to Claim 32, for the preparation of a medicament intended for the preventive or curative treatment of genetic diseases such as haemophilia, diabetes, cystic fibrosis, Duchenne's or Becker's myopathy, autoimmune diseases or acquired diseases such as cancers, tumors, cardiovascular diseases, diseases of infectious origin such as hepatitis B or C and AIDS by transfer and expression of a gene of interest in a host cell or organism.

37. Use of an adenoviral vector according to one of Claims 1 to 10 and 14 to 18, of a viral preparation according to Claim 30 or 31 or of a host cell according to Claim 33, for the preparation of a medicament intended for the treatment of diseases by gene therapy or immunotherapy.
